# EUROPEAN PATENT APPLICATION

(11) **EP 4 589 598 A1**
(43) Date of publication of application: **23.07.2025**
(21) Application number: 25152082.1
(22) Date of filing: 15.01.2025
(51) Int. Cl.: G16C 20/30, G16C 60/00

(54) **MACHINE LEARNING TECHNOLOGIES FOR ASSESSING THERMAL ENDURANCE CHARACTERISTICS OF PHYSICAL MATERIALS**

(30) Priority: 19.01.2024 US 202418418170
(71) Applicant: UL LLC, Northbrook, IL 60062 (US)
(72) Inventor: O'SHEA, Daniel, Northbrook, 60062 (US); MITRA, Shubhankar, Northbrook, 60062 (US); STREET, Stephen, Northbrook, 60062 (US); MEHDIYEV, Rashid, Northbrook, 60062 (US); GUPTA, Aniruddha, Northbrook, 60062 (US); ANDRICK, Raymond, Northbrook, 60062 (US); LIN, Alice Ichun, Northbrook, 60062 (US); AGHAJARIAN, Mickael, Chicago, IL 60611 (US)
(74) Representative: Maiwald GmbH

(57) **Abstract**

Systems and methods for using machine learning to assess thermal endurance characteristics, such as relative thermal indices (RTIs), for materials are disclosed. According to certain aspects, an electronic device may train a machine learning model using a set of training data that comprises coordinates indicative of a plurality of characteristics for a plurality of materials, and is labeled with at least one thermal endurance characteristic for each of the plurality of materials. The trained machine learning model may analyze a set of coordinates associated with a candidate material, and output at least one thermal endurance characteristic for that candidate material. The machine learning model may also be continuously updated and used in subsequent analyses.

## Description

### FIELD

The present disclosure is directed to improvements related to assessing characteristics of physical materials. More particularly, the present disclosure is directed to platforms and technologies for using machine learning techniques to assess thermal endurance characteristics of physical materials.

### BACKGROUND

Long term thermal aging (LTTA) is a test process used to study the effects of extended exposure to elevated temperatures on materials. It involves subjecting a material to higher temperatures for an extended period of time, often months or even years, to simulate the long-term aging that the material might experience in real-world conditions. This process helps researchers and engineers understand how properties, performance, and structural integrity of a given material change over time due to thermal stresses and environmental factors. Generally, LTTA is used to determine the thermal endurance characteristics for a given material that quantifies the thermal aging resistance of that material.

However, LTTA is a time-consuming process that requires ongoing resources and monitoring, often for months or years. This results in higher costs and longer research timelines, making it less feasible for various industries, particularly those with fast-paced development cycles. While LTTA aims to simulate real-world conditions, certain accelerated aging methods that subject materials to higher temperatures for shorter periods are sometimes used to speed up the testing process. However, these methods may over-accelerate the characteristics under evaluation resulting in mechanisms not relevant to thermal performance at service temperatures, which leads to discrepancies and inaccuracies in the results. Additionally, for some industries, subjecting materials to LTTA might not be practical due to ethical concerns or regulatory limitations, especially if the tested materials are intended for critical applications where rapid development and testing are necessary.

Accordingly, there is an opportunity for platforms and technologies to efficiently, accurately, and effectively assess thermal endurance characteristics of physical materials.

### SUMMARY

In an embodiment, a computer-implemented method of using machine learning to assess thermal endurance characteristics of materials is provided. The computer-implemented method may include: training, by at least one processor, a machine learning model using a set of training data, wherein the set of training data (i) comprises training sets of coordinates indicative of a plurality of characteristics for a plurality of materials, and (ii) is labeled with at least one thermal endurance characteristic for each material of the plurality of materials, wherein the at least one thermal endurance characteristic is associated with each training set of coordinates, of the training sets of coordinates, for that material; accessing, by the at least one processor, sets of coordinates indicative of the plurality of characteristics for a candidate material; analyzing, by the at least one processor using the machine learning model that was trained, the sets of coordinates indicative of the plurality of characteristics for the candidate material; and based on the analyzing, outputting, by the machine learning model, at least one predicted thermal endurance characteristic for the candidate material.

In another embodiment, a system for using machine learning to assess thermal endurance characteristics of materials is provided. The system comprises: a memory storing a set of computer-readable instructions; and one or more processors interfaced with the memory. The one or more processors may be configured to execute the set of computer-readable instructions to cause the one or more processors to: train a machine learning model using a set of training data, wherein the set of training data (i) comprises training sets of coordinates indicative of a plurality of characteristics for a plurality of materials, and (ii) is labeled with at least one thermal endurance characteristic for each material of the plurality of materials, wherein the at least one thermal endurance characteristic is associated with each training set of coordinates, of the training sets of coordinates, for that material, access a set of coordinates indicative of the plurality of characteristics for a candidate material, analyze, using the machine learning model that was trained, the set of coordinates indicative of the plurality of characteristics for the candidate material, and based on the analyzing, output, by the machine learning model, at least one predicted thermal endurance characteristic for the candidate material.

Further, in an embodiment, a non-transitory computer-readable storage medium configured to store instructions executable by one or more processors is provided. The instructions may include: instructions for training a machine learning model using a set of training data, wherein the set of training data (i) comprises training sets of coordinates indicative of a plurality of characteristics for a plurality of materials, and (ii) is labeled with at least one thermal endurance characteristic for each material of the plurality of materials, wherein the at least one thermal endurance characteristic is associated with each training set of coordinates, of the training sets of coordinates, for that material; instructions for accessing a set of coordinates indicative of the plurality of characteristics for a candidate material; instructions for analyzing, using the machine learning model that was trained, the set of coordinates indicative of the plurality of characteristics for the candidate material; and instructions for, based on the analyzing, outputting, by the machine learning model, at least one predicted thermal endurance characteristic for the candidate material.

### BRIEF DESCRIPTION OF THE FIGURES

FIG. 1 depicts an overview of components and entities associated with the systems and methods, in accordance with some embodiments.
FIG. 2 depicts an overview of certain components configured to facilitate the systems and methods, in accordance with some embodiments.
FIGs. 3A-3C depict example graphs indicating various properties of an example material, in accordance with some embodiments.
FIG. 4 depicts an example diagram indicating various functionalities of the systems and methods, in accordance with some embodiments.
FIG. 5 illustrates an example flow diagram of using machine learning to determine RTIs for materials, in accordance with some embodiments.
FIG. 6 is an example hardware diagram of a server configured to perform various functionalities, in accordance with some embodiments.

### DETAILED DESCRIPTION

The LTTA test method is a process used to assess the stability and performance of materials (e.g., polymers) over an extended period of time under elevated temperatures. This test is commonly employed in industries such as automotive, aerospace, electrical equipment and electronics (e.g., home appliances, consumer electronics, industrial controls, electrical accessories), and construction, where certain materials present in parts and other components need to maintain their properties over prolonged exposure to heat. The LTTA test is crucial for evaluating the long-term durability and reliability of materials, as well as predicting potential changes in their mechanical, electrical, flammability, and other critical properties.

A relative thermal index (RTI) is a thermal endurance characteristic of a material which is an indication of the ability of the material to retain a particular property (physical, electrical, etc.) when exposed to elevated temperatures for an extended period of time. It is the maximum temperature below which a material maintains its characteristics over a reasonable period of time. For each material, a number of relative thermal indices can be established, where each index is related to a specific property and a specific thickness of the material. Stated differently, a relative thermal index is the maximum temperature below which a material maintains its characteristics over a reasonable period.

Generally, the LTTA test involves subjecting test specimens or samples of a given material to elevated temperatures for an extended duration. A set of samples for a material to be tested is prepared and checked for uniformity and the presence of any defects. These specimens are usually in the form of standardized test specimen shapes depending on the material type, its furnished form (e.g., molding resins, sheets, films, or other relevant shapes), and its intended application. The selected temperature for the test is usually higher than the expected service temperature of the material, and the selected duration of the test can range from months to years, depending on the intended application and the desired level of aging to be simulated.

Generally, the specimens are placed in a controlled environment, such as an oven or environmental chamber, that can maintain a constant temperature over the course of the test. The specimens are then subjected to the elevated temperature for the predetermined duration This allows the material to undergo thermal aging and the associated chemical reactions and physical changes to occur. Such experimental processes simulate the effects of prolonged exposure to high temperatures on the material in real-world conditions. Throughout the test period, the specimens are periodically evaluated to measure changes in their properties, which may include testing mechanical properties like tensile strength, elongation, and impact resistance, electrical properties like dielectric strength, and other properties like flammability.

The resulting output of the LTTA test includes data on how certain properties of the material have changed over time and under the specified temperature conditions. This data can be used to assess the long-term stability of the material, predict its behavior in real-world applications, and make informed decisions about its suitability for specific use cases. The output might be presented as graphs showing property changes over time or tables summarizing the observed alterations in mechanical strength, mechanical impact, electrical, and flammability characteristics.

In particular, an output of the LTTA test for a given material (e.g., a plastic or polymeric material that may be used in electrical equipment of electronics) is the RTI that quantifies the thermal aging resistance of that material. Generally, the RTI is a measure of the maximum continuous use temperature of a material, and it is defined as the highest temperature at which the material can be used in a specific application while maintaining its essential properties, such as mechanical strength, electrical insulation capabilities, and other relevant characteristics. The RTI for a given material may be determined based on the retention of specific properties after thermal aging. For instance, if a material is subjected to an LTTA test and its mechanical strength, electrical insulation capabilities, and other relevant properties are still within acceptable limits after the aging process, the RTI can be defined as the maximum temperature at which these properties were retained. The RTI value thus serves as a critical indicator for manufacturers and engineers to determine the appropriate maximum operating temperature for components (e.g., enclosures and switches) made from a particular material, and it helps ensure that the components will remain functional and safe over their intended service life, even when exposed to elevated temperatures.

The RTI value is expressed as a temperature in degrees Celsius (°C) or Fahrenheit (°F). Different materials and types of materials may be tested to ascertain respective RTIs including, for example, thermoplastics, thermosets, reinforced plastics, elastomers, composites, and/or the like.

Generally, the thermal endurance characteristic of a material may be divided into three sub-categories: electrical RTI, mechanical impact RTI, and mechanical strength RTI. Electrical RTI focuses on the ability of insulating materials to maintain their electrical insulation properties when exposed to elevated temperatures. This sub-category considers factors such as dielectric strength, resistivity, and breakdown voltage, where electrical RTI indicates the highest temperature at which the material can function as an effective electrical insulator without significant degradation in its electrical properties. It is a useful parameter for materials used in electrical devices and other components where reliable electrical insulation is essential.

Mechanical impact RTI assesses the ability of a given material to withstand mechanical stresses, such as impact or shock, at elevated temperatures. This sub-category may be particularly relevant for materials used in applications where impact resistance is vital, such as in enclosures, housings, or structural components, where the mechanical impact RTI provides information about the toughness and durability of the material under dynamic conditions and high temperatures.

Mechanical strength RTI focuses on the ability of a given material to retain its mechanical strength and structural integrity when exposed to elevated temperatures. It measures properties like tensile strength and flexural strength after thermal aging, and indicates the maximum temperature at which the material can maintain its structural integrity without significant loss in mechanical properties. This parameter is important for materials used in load-bearing applications, where maintaining strength and structural stability is crucial.

However, as discussed herein, there are several downsides and challenges associated with LTTA testing. In particular, these tests are time-consuming and costly, and require the availability of large quantities of the material to be tested over the long duration. Additionally, it is difficult to accurately predict actual real-world conditions, as LTTA tests are typically conducted under controlled laboratory conditions, and the test conditions may not always accurately represent the multiple stresses the material will face in the actual application.

Further, LTTA tests may not always account for all factors that can influence material degradation, as some degradation mechanisms may not become apparent until after years of exposure, leading to inaccuracies in RTI assessments. Additionally, for newly-developed materials, there may be insufficient data available on their long-term behavior and organizations may need to rely on accelerated aging tests or other methods to make initial assessments, the results of which are inaccurate. Moreover, selecting the appropriate aging conditions (e.g., temperature, humidity, etc.) for LTTA tests is challenging and requires significant prior knowledge or preliminary experiments to establish suitable conditions.

The present embodiments employ machine learning techniques to determine the thermal endurance characteristics for materials without needing to conduct full LTTA testing. According to aspects, an electronic device may train a machine learning model using a set of training data that includes coordinates indicating a plurality of characteristics for a plurality of materials and is labeled with at least one RTI for each of the plurality of materials. Additionally, a test set of coordinates for a candidate material is input into the machine learning model which analyzes the test set of coordinates and outputs at least one predicted RTI for the candidate material.

The described systems and methods improve on existing technologies, namely technologies for assessing RTIs for materials. In particular, the systems and methods can provide accurate results much faster than physical testing, which is especially advantageous when making shorter design and material selection decisions. Furthermore, the systems and methods significantly reduce costs associated with physical LTTA tests, which have extensive durations, resource requirements, and equipment maintenance.

The systems and methods additionally improve on existing machine learning technologies. In particular, the training data for the machine learning model includes training sets of coordinates that are indicative of a plurality of characteristics of given materials, including an infrared analysis, a thermogravimetric analysis, and a differential scanning calorimetry. These sets of coordinates are generally used to assess various characteristics of materials, including chemical composition, quality control, thermal stability, composition changes, thermal properties, and purity assessment; however, these sets of coordinates are neither available nor used in conventional LTTA tests. Thus, by training the machine learning model using this unique dataset, the systems and methods allow for the incorporation of richer and wider datasets, thus enabling the machine learning model to learn complex mappings between multiple input data types and test outcomes.

Additionally, the trained machine learning model is able to input similarly-formatted data (i.e., coordinates related to an infrared analysis, a thermogravimetric analysis, and a differential scanning calorimetry) for a candidate material, analyze the data, and output predicted test results, which are impossible to accurately gain using conventional LTTA testing. By incorporating multi-format datasets, the systems and methods represent an improvement even on conventional machine learning models trained only on limited testing data. Moreover, any modifications to the data output by the machine learning model may be re-input into the machine learning model, which results in improved output on subsequent analyses by the machine learning model.

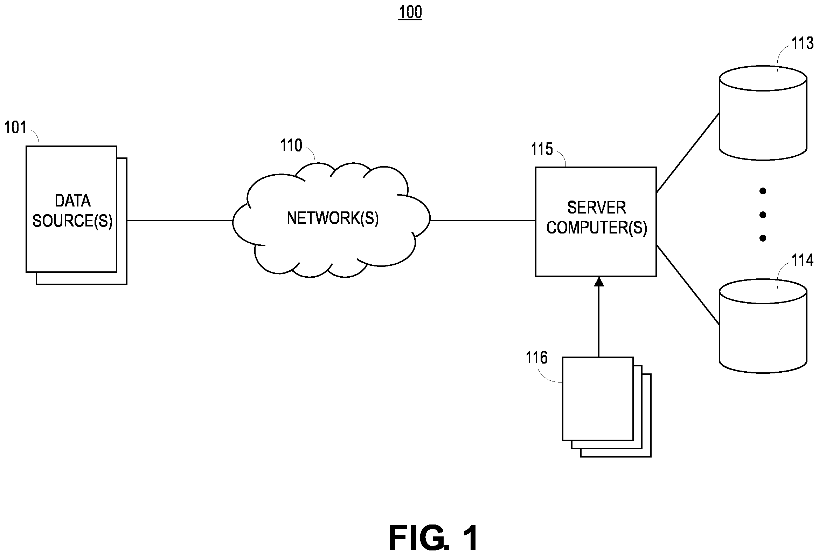

FIG. 1 illustrates an overview of a system 100 of components configured to facilitate the described systems and methods. It should be appreciated that the system 100 is merely an example and that alternative or additional components are envisioned.

As illustrated in FIG. 1, the system 100 may include a set of data sources 101 such as electronic devices, databases, or other types of data sources. According to embodiments, the set of data sources 101 may be an electronic device such as a mobile device (e.g., a smartphone), desktop computer, notebook computer, tablet, phablet, GPS (Global Positioning System) or GPS-enabled device, smart watch, smart glasses, smart bracelet, wearable electronic, PDA (personal digital assistant), pager, computing device configured for wireless communication, and/or the like, where the electronic device may be associated with an individual or an entity such as a company, business, corporation, or the like (e.g., a server computer or machine).

The set of data sources 101 may communicate with a server computer(s) 115 via one or more networks 110. In embodiments, the network(s) 110 may support any type of data communication via any standard or technology (e.g., GSM, CDMA, VoIP, TDMA, WCDMA, LTE, EDGE, OFDM, GPRS, EV-DO, UWB, Internet, IEEE 802 including Ethernet, WiMAX, Wi-Fi, Bluetooth, 4G/5G/6G, Edge, and others). The server computer(s) 115 may be associated with an entity such as a company, business, corporation, or the like (generally, a company), and may be configured to employ techniques to train machine learning models and use the machine learning models to determine RTIs for materials. The server computer(s) 115 may include various components that support communication with the set of data sources 101.

According to embodiments, the server computer(s) 115 may access a set(s) of data 116 which may include a set(s) of training data as well as a set(s) of validation data. The server computer(s) 115 may train a machine learning model using the set(s) of data 116. In particular, the server computer 115 may initially train a set of machine learning models using the set(s) of training data and then apply or input the set(s) of validation data into a set of generated machine learning models to determine which of the machine learning models is most accurate or otherwise may be used as the final or selected machine learning model.

Additionally, the set of data sources 101 may compile, store, access, and/or avail data or information associated with different materials. In particular, the data or information may indicate properties of different materials, and may include sets of coordinates indicative of an infrared analysis, a thermogravimetric analysis, and/or a differential scanning calorimetry for the different materials. The server computer(s) 115 may analyze this data/information using the trained machine learning model according to the functionalities as described herein, which may result in one or more predicted RTIs for a given material. In some implementations, the server computer 115 may access the raw data or information (and/or the set(s) of data 116) from the set of data sources 101, or from another source.

The server computer(s) 115 may be configured to interface with or support a memory or storage 113, 114 capable of storing various data, such as in one or more databases or other forms of storage. According to embodiments, the storage 113, 114 may store data or information associated with any machine learning model(s) that is generated by the server computer(s) 115. Additionally, the server computer(s) 115 may access, from the storage 113, 114, data associated with the stored machine learning model to input a set of inputs into the machine learning model. Further, the storage 113, 114 may store data associated with materials, such as data indicative of the properties of the materials.

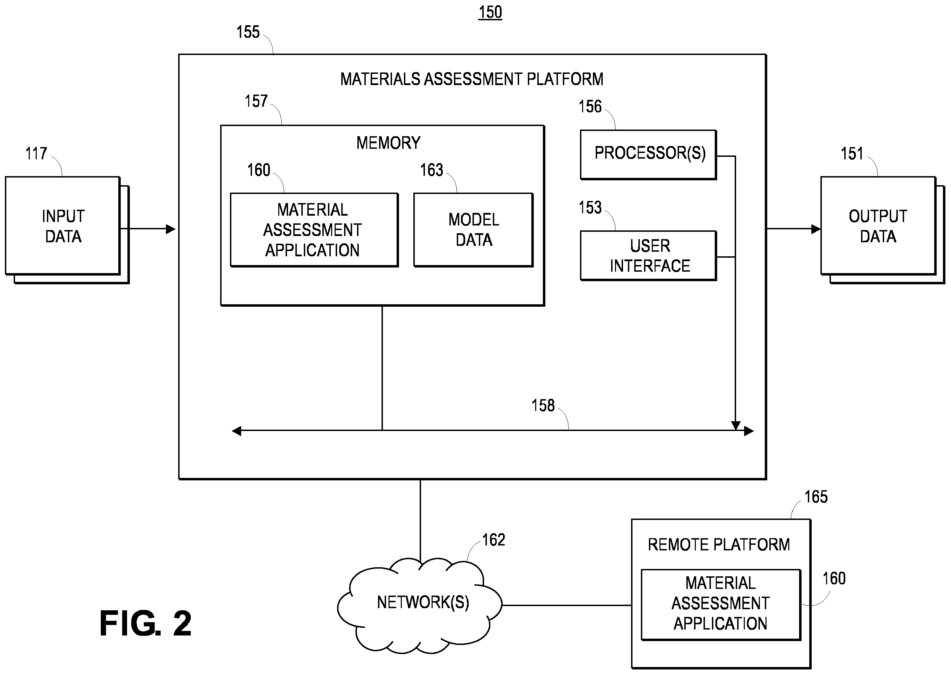

Although depicted as a single server computer 115 in FIG. 1, it should be appreciated that the server computer(s) 115 may be in the form of a distributed cluster of computers, servers, machines, cloud-based services, or the like. In this implementation, an accessing entity may utilize the distributed server computer(s) 115 as part of an on-demand cloud computing platform. Accordingly, when the set of data sources 101 interface with the server computer(s) 115, the set of data sources 101 may actually interface with one or more of a number of distributed computers, servers, machines, or the like, to facilitate the described functionalities. Further, it should be appreciated that varying amounts of the set of data sources 101 and the storage 113, 114 amounts are envisioned. FIG. 2 depicts more specific components associated with the systems and methods.

FIG. 2 an example environment 150 in which input data 117 is processed into output data 151 via a materials assessment platform 155, according to embodiments. The materials assessment platform 155 may be implemented on any computing device or combination of computing devices, including the server computer(s) 115 as discussed with respect to FIG. 1. Components of the computing device may include, but are not limited to, a processing unit (e.g., processor(s) 156), a system memory (e.g., memory 157), and a system bus 158 that couples various system components including the memory 157 to the processor(s) 156. In some embodiments, the processor(s) 156 may include one or more parallel processing units capable of processing data in parallel with one another. The system bus 158 may be any of several types of bus structures including a memory bus or memory controller, a peripheral bus, or a local bus, and may use any suitable bus architecture. By way of example, and not limitation, such architectures include the Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus (also known as Mezzanine bus).

The materials assessment platform 155 may further include a user interface 153 configured to present content (e.g., input data, output data, processing data, and/or other information). Additionally, a user may review results of a material assessment and make selections to the presented content via the user interface 153, such as to review output data presented thereon, make selections, and/or perform other interactions. The user interface 153 may be embodied as part of a touchscreen configured to sense touch interactions and gestures by the user. Although not shown, other system components communicatively coupled to the system bus 158 may include input devices such as cursor control device (e.g., a mouse, trackball, touch pad, etc.) and keyboard (not shown). A monitor or other type of display device may also be connected to the system bus 158 via an interface, such as a video interface. In addition to the monitor, computers may also include other peripheral output devices such as a printer, which may be connected through an output peripheral interface (not shown).

The memory 157 may include a variety of computer-readable media. Computer-readable media may be any available media that can be accessed by the computing device and may include both volatile and nonvolatile media, and both removable and non-removable media. By way of non-limiting example, computer-readable media may comprise computer storage media, which may include volatile and nonvolatile, removable and non-removable media implemented in any method or technology for storage of information such as computer-readable instructions, routines, applications (e.g., a material assessment application 160), data structures, program modules or other data. Computer storage media may include, but is not limited to, RAM, ROM, EEPROM, FLASH memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can accessed by the processor 156 of the computing device.

The materials assessment platform 155 may operate in a networked environment and communicate with one or more remote platforms, such as a remote platform 165, via a network(s) 162, such as a local area network (LAN), a wide area network (WAN), or other suitable network. The platform 165 may be implemented on any computing device and may include many or all of the elements described above with respect to the platform 155. In some embodiments, the material assessment application 160 as will be further described herein may be stored and executed by the remote platform 165 instead of by or in addition to the platform 155.

Generally, each of the input data 117 and the output data 151 may be embodied as any type of electronic document, file, template, etc., that may include various graphical/visual and/or textual content, and may be stored in memory as program data in a hard disk drive, magnetic disk and/or optical disk drive in the materials assessment platform 155 and/or the remote platform 165. The materials assessment platform 155 may support one or more techniques, algorithms, or the like for analyzing the input data 117 to generate the output data 151. In particular, the material assessment application 160 may train and use a machine learning model to assess RTIs for given candidate materials. The memory 157 may store the output data 151 and other data that the materials assessment platform 155 generates or uses in associated with the analysis of the input data 117.

According to embodiments, the material assessment application 160 may employ machine learning and artificial intelligence techniques such as, for example, a regression analysis (e.g., a logistic regression, linear regression, random forest regression, probit regression, or polynomial regression), entity resolution, classification analysis, k-nearest neighbors, decisions trees, random forests, boosting, neural networks, support vector machines, deep learning, reinforcement learning, Bayesian networks, or the like. When the input data 117 is a training dataset(s), the material assessment application 160 may analyze/process the input data 117 to generate a machine learning model(s) for storage as part of model data 163 that may be stored in the memory 157. In embodiments, various of the output data 151 may be added to the machine learning model(s) stored as part of the model data 163. In analyzing or processing the input data 117, the material assessment application 160 may use any of the output data 151 previously generated by the materials assessment platform 155.

The material assessment application 160 (or another component) may cause the output data 151 (and, in some cases, the training or input data 117) to be displayed on the user interface 153 for review by the user of the materials assessment platform 155, such as to review a certain model input and/or results of the machine learning analyses, as part of a dashboard, interface, or the like. The user may select to review and/or modify the displayed data. For instance, the user may review the output data 151 to manually override model outputs, add additional outputs, and/or facilitate other functionalities.

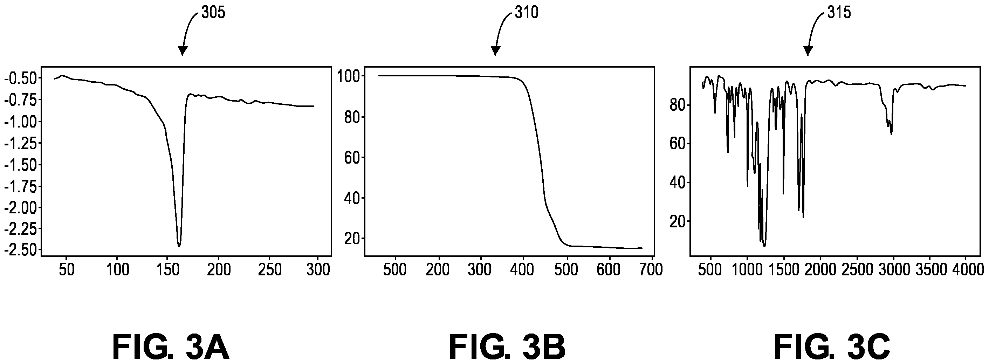

FIGs. 3A-3C illustrate example graphs indicating various properties of an example material. It should be appreciated that each of the respective graphs of FIGs. 3A-3C are exemplary, and that different materials have different values for the various properties.

FIG. 3A illustrates a graph 305 depicting a Differential Scanning Calorimetry (DSC) curve for a material, which may measure the heat flow into or out of the material as it undergoes temperature changes, revealing phase transitions, crystallization, and thermal properties, among other properties.

In the graph 305 of FIG. 3A, the DSC results are plotted with temperature (°C) on the x-axis and heat flow (mW or mJ) on the y-axis. Endothermic and exothermic events related to phase transitions, crystallization, and chemical reactions may be observed as peaks and troughs in the DSC curve. For example, as illustrated in FIG. 3A, at a temperature around 160°C, the material experiences a phase transition.

FIG. 3B illustrates a graph 310 depicting a Thermogravimetric Analysis (TGA) curve for a material, which may measure the change in weight of a material as it is subjected to increasing temperature. The TGA curve may enable for the identification of the individual thermal stability and decomposition properties of various components of the material formulation, and the presence of volatile components.

In the graph 310 of FIG. 3B, the TGA results are plotted with temperature (°C) on the x-axis and weight percentage (or mass) on the y-axis (i.e., the weight percentage or mass of the material remaining as a function of temperature). The TGA curve may show weight loss corresponding to the release of volatile components or decomposition of individual components of the material. For example, as illustrated in FIG. 3B, at a temperature around 400°C, the material begins to rapidly decompose.

FIG. 3C illustrates a graph 315 depicting an Infrared Analysis (IR) curve for a material, which measures the absorption of infrared light by the material. The curve may provide information about the molecular vibrations and functional groups present in the material.

In the graph 315 of FIG. 3C, the IR results are plotted with Wavenumber (cm^-1) (which is inversely proportional to the wavelength of the infrared light) on the x-axis and percent transmittance or absorbance on the y-axis, resulting in an IR spectrum. Different functional groups in the material may absorb infrared light at specific wavenumbers, creating characteristic peaks in the spectrum. The y-axis depicts the intensity of the absorbed light, either in terms of percent transmittance (i.e., how much light passes through the material) or absorbance (i.e., how much light is absorbed by the material).

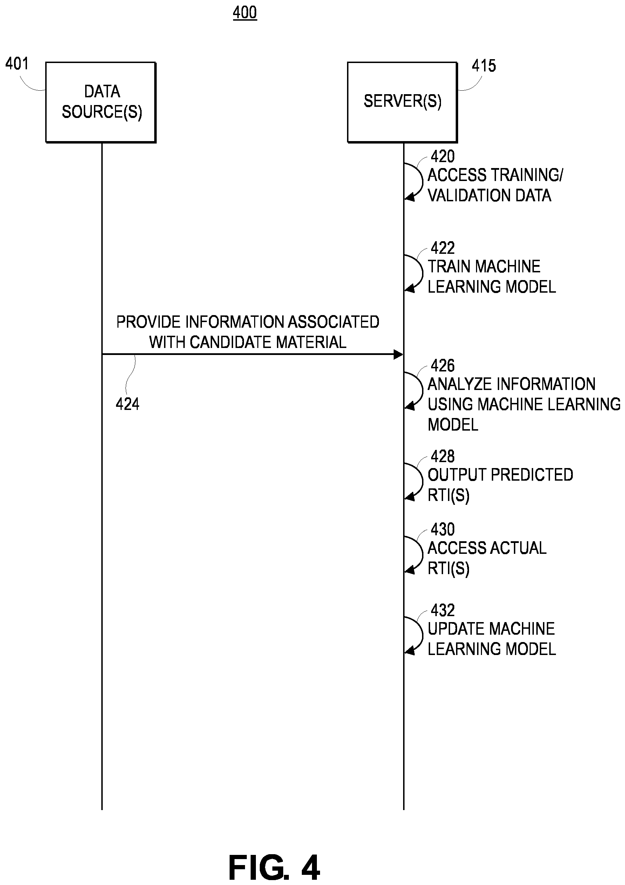

FIG. 4 illustrates an example diagram 400 associated with the described embodiments. The diagram 400 includes a data source(s) 401 (such as the data source(s) 101 as described with respect to FIG. 1) and a server(s) 415 (such as the server(s) 115 as described with respect to FIG. 1). It should be appreciated that the functionalities are merely exemplary, and the additional or alternative functionalities, in differing orders, are envisioned.

As illustrated in FIG. 4, the server(s) 415 may be configured to access (420) training and validation datasets. According to embodiments, each training and validation dataset may include sets of coordinates respectively indicative of a plurality of characteristics for a given material, where the plurality of characteristics may be an infrared analysis, a thermogravimetric analysis, and a differential scanning calorimetry for that given material, as described with respect to FIGs. 3A-3C. It should be appreciated that a given set of coordinates may be (x,y) coordinates of a given curve, such as the infrared analysis, a thermogravimetric analysis, and differential scanning calorimetry curves as illustrated in FIGs. 3A-3C.

Additionally, each training and validation dataset may be labeled with at least one thermal endurance characteristic for that associated material. According to embodiments, the at least one thermal endurance characteristic may be one or more of an electrical RTI, a mechanical impact RTI, or a mechanical strength RTI. For example, example sets of coordinates for a given plastic material may include three (3) separate (x,y) coordinate plots for the infrared analysis, the thermogravimetric analysis, and the differential scanning calorimetry for that given plastic material, where the sets of coordinates may be labeled with each of an electrical RTI, a mechanical impact RTI, and a mechanical strength RTI. It should be appreciated that a given sets of coordinates may be labeled with one or more of the electrical RTI, mechanical impact RTI, or mechanical strength RTI.

The server(s) 415 may train (422) a machine learning model using the training and validation datasets. According to embodiments, the server(s) 415 may be configured to preprocess the training and validation datasets by cleaning, feature engineering, and/or transforming the raw data into a format suitable for machine learning. Generally, the server(s) 415 may use the training dataset to train the model, and the validation dataset to fine-tune the model and monitor its performance.

In embodiments, the server(s) 415 may determine or receive instructions related to the type of machine learning model to use. For example, different machine learning models may be a decision tree, a neural network, a support vector machine, or any other suitable model. During training, the model may learn the underlying patterns and relationships in the training dataset. The server(s) 415 may iteratively update any parameters or weights of the model to minimize a chosen loss function, which may measure any difference between the predictions of the model and actual target values in the training dataset.

Additionally, the server(s) 415 may adjust hyperparameters that are not learned from the data, such as learning rate, depth of a decision tree, number of hidden layers in a neural network, and/or others. The server(s) 415 may use different hyperparameters in tuning the model, and may evaluate the performance of the model on the validation dataset to select the best hyperparameters.

Periodically or after a set number of training iterations (i.e., epochs), the server(s) 415 may assess the performance of the model using the validation dataset, such as by evaluating accuracy, F1 score, mean squared error, or others. Generally, the performance of the model on the validation dataset helps to detect issues like overfitting (i.e., the model is too complex and performs well on training data but poorly on unseen data) or underfitting (i.e., the model is too simple and performs poorly on both training and validation data).

Based on the results of the validation, the server(s) 415 may fine-tune the model further by adjusting hyperparameters or making changes to the model architecture. Generally, the steps of training, validation, and fine-tuning the model are typically repeated until the performance of the model on the validation dataset reaches a satisfactory level. In embodiments, and at this point, the server(s) 415 may evaluate the performance of the model on a separate test dataset, which may provide a more realistic estimate of how the model will perform on unseen data. After testing the machine learning model, the machine learning model may be used to analyze data associated with a candidate material.

In particular, the data source(s) 401 may provide (424) information associated with a candidate material to the server(s) 415. It should be appreciated that the server(s) 415 may locally access this information, or receive the information from another source. In embodiments, the information may include sets of coordinates indicative of an infrared analysis, a thermogravimetric analysis, and a differential scanning calorimetry for the candidate material. For example, the information may include a first set of (x,y) coordinates for the infrared analysis, a second set of (x,y) coordinates for the thermogravimetric analysis, and a third set of (x,y) coordinates for the differential scanning calorimetry, as similarly discussed herein with respect to FIGs. 3A-3C.

The server(s) 415 may analyze (426) the information using the machine learning model. In particular, the server(s) 415 may input, into the trained machine learning model, the sets of coordinates indicative of the infrared analysis, the thermogravimetric analysis, and the differential scanning calorimetry for the candidate material.

Based on the analyzing in (426), the server computer(s) 415 may output (428), via the machine learning model, at least one predicted thermal endurance characteristic for the candidate material. In embodiments, the at least one predicted thermal endurance characteristic may be at least one of: an electrical relative thermal index (RTI), a mechanical impact RTI, or a mechanical strength RTI. Additionally, the machine learning model may output a confidence level for each of the at least one predicted thermal endurance characteristic for the candidate material. In embodiments, the confidence level may be a percentage that may range from 0% to 100%, or may be another metric indicating the likelihood that a given predicted thermal endurance characteristic accurately reflects the actual thermal endurance characteristic for the candidate material.

The server(s) 415 may access (430) at least one actual RTI for the candidate material. In embodiments, the server(s) 415 may receive or otherwise access, as an input (e.g., via a user interface), the at least one actual RTI, which may be derived from a physical test of the candidate material, or via another channel. The server(s) 415 may additionally update (432) the machine learning model using information associated with the candidate material (i.e., the sets of coordinates indicative of the plurality of characteristics for the candidate material) and the at least one actual thermal endurance characteristic for the candidate material. Accordingly, the server(s) 415 may continuously update the machine learning model as new or updated data is accessed or determined. Further, the server(s) 415 may use the updated machine learning model on subsequent analysis, which results in improved accuracy for the outputs of the machine learning model.

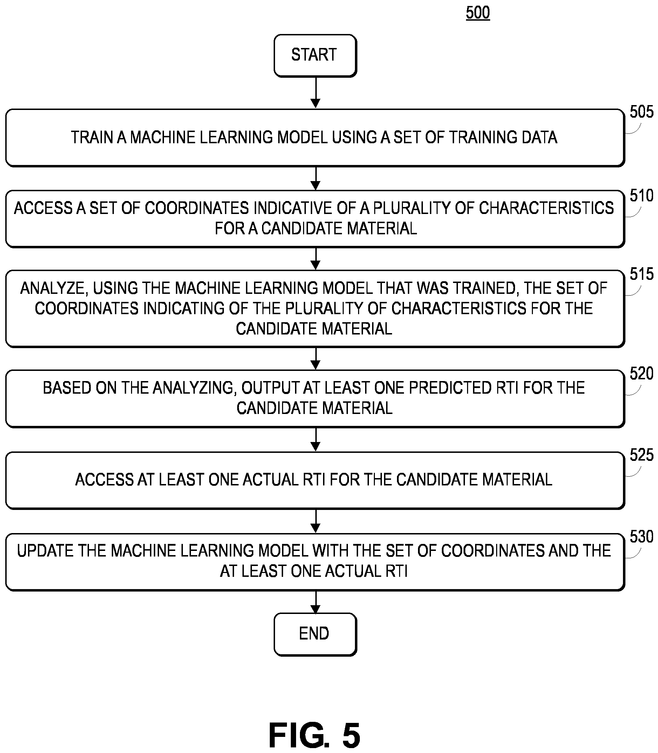

FIG. 5 is a block diagram of an example method 500 of using machine learning to assess thermal endurance characteristics of materials. The method 500 may be facilitated by one or more electronic devices (such as the server computer 115 as depicted in FIG. 1).

The method 500 may begin at block 505 when the electronic device(s) trains a machine learning model using a set of training data. According to embodiments, the set of training data may (i) comprise training sets of coordinates indicative of a plurality of characteristics for a plurality of materials, and (ii) be labeled with at least one thermal endurance characteristic for each material of the plurality of materials, wherein the at least one thermal endurance characteristic may be associated with each training set of coordinates, of the training sets of coordinates, for that material.

Further, in embodiments, the machine learning model may be trained using the set of training data and a set of validation data, wherein the set of validation data may (i) comprise validation sets of coordinates indicative of the plurality of characteristics for the plurality of materials, and (ii) be labeled with the at least one thermal endurance characteristic for each material of the plurality of materials, wherein the at least one thermal endurance characteristic is associated with each validation set of coordinates, of the validation sets of coordinates, for that material. Additionally, in embodiments, the at least one thermal endurance characteristic may be one or more of an electrical RTI, a mechanical impact RTI, or a mechanical strength RTI.

The electronic device may access (block 510) a set of coordinates indicative of the plurality of characteristics for a candidate material. In embodiments, the set of coordinates may be indicative of at least one of: an infrared analysis, a thermogravimetric analysis, or a differential scanning calorimetry for the candidate material.

The electronic device may analyze (block 515), using the machine learning model that was trained, the set of coordinates indicating the plurality of characteristics for the candidate material, such as by inputting the set of coordinates into the trained machine learning model. Further, at block 520, the machine learning model may, based on the analyzing, output at least one predicted thermal endurance characteristic for the candidate material, such as one or more of an electrical RTI, a mechanical impact RTI, or a mechanical strength RTI. In embodiments, the machine learning model may additionally output a confidence level for each of the at least one predicted thermal endurance characteristic for the candidate material.

The electronic device may access (block 525) at least one actual thermal endurance characteristic (e.g., an actual RTI) for the candidate material, such as the result of physically testing the candidate material or via another channel. Additionally, the electronic device may update (block 530) the machine learning model with the set of coordinates indicative of the plurality of characteristics for the candidate material, and the at least one actual thermal endurance characteristic for the candidate material.

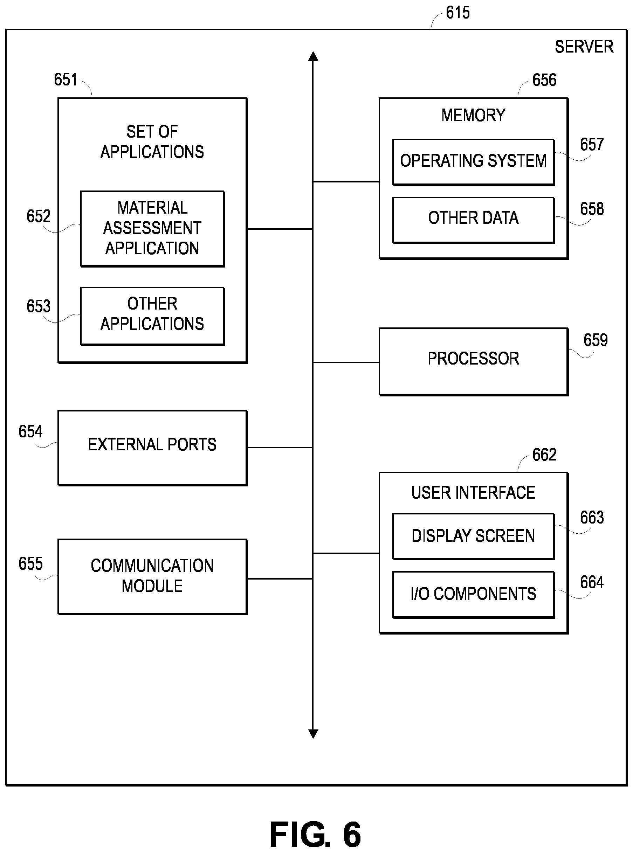

FIG. 6 illustrates a hardware diagram of an example server 615 (e.g., the server 115 as described with respect to FIG. 1), in which the functionalities as discussed herein may be implemented. It should be appreciated that the components of the server 615 are merely exemplary, and that additional or alternative components and arrangements thereof are envisioned.

The server 615 may include a processor 659 as well as a memory 656. The memory 656 may store an operating system 657 capable of facilitating the functionalities as discussed herein as well as a set of applications 651 (i.e., machine readable instructions). For example, one of the set of applications 651 may be a material assessment application 652, such as to train machine learning models and use the machine learning models to assess RTIs for materials. It should be appreciated that one or more other applications 653 are envisioned.

The processor 659 may interface with the memory 656 to execute the operating system 657 and the set of applications 651. According to some embodiments, the memory 656 may also store other data 658, such as machine learning model data and/or other data such as data related to materials that may be used in the training, analyses, and determinations as discussed herein. The memory 656 may include one or more forms of volatile and/or nonvolatile, fixed and/or removable memory, such as read-only memory (ROM), electronic programmable read-only memory (EPROM), random access memory (RAM), erasable electronic programmable read-only memory (EEPROM), and/or other hard drives, flash memory, MicroSD cards, and others.

The server 615 may further include a communication module 655 configured to communicate data via one or more networks (not shown in FIG. 6). According to some embodiments, the communication module 655 may include one or more transceivers (e.g., WAN, WWAN, WLAN, and/or WPAN transceivers) functioning in accordance with IEEE standards, 3GPP standards, or other standards, and configured to receive and transmit data via one or more external ports 654.

The server 615 may further include a user interface 662 configured to present information to a user and/or receive inputs from the user. As shown in FIG. 6, the user interface 662 may include a display screen 663 and I/O components 664 (e.g., ports, capacitive or resistive touch sensitive input panels, keys, buttons, lights, LEDs, external or built in keyboard). According to some embodiments, the user may access the server 615 via the user interface 662 to review information, make selections, and/or perform other functions.

In some embodiments, the server 615 may perform the functionalities as discussed herein as part of a "cloud" network or may otherwise communicate with other hardware or software components within the cloud to send, retrieve, or otherwise analyze data.

In general, a computer program product in accordance with an embodiment may include a computer usable storage medium (e.g., standard random access memory (RAM), an optical disc, a universal serial bus (USB) drive, or the like) having computer-readable program code embodied therein, wherein the computer-readable program code may be adapted to be executed by the processor 659 (e.g., working in connection with the operating system 657) to facilitate the functions as described herein. In this regard, the program code may be implemented in any desired language, and may be implemented as machine code, assembly code, byte code, interpretable source code or the like (e.g., via Golang, Python, Scala, C, C++, Java, Actionscript, Objective-C, Javascript, CSS, XML). In some embodiments, the computer program product may be part of a cloud network of resources.

Although the following text sets forth a detailed description of numerous different embodiments, it should be understood that the legal scope of the invention may be defined by the words of the claims set forth at the end of this patent. The detailed description is to be construed as exemplary only and does not describe every possible embodiment, as describing every possible embodiment would be impractical, if not impossible. One could implement numerous alternate embodiments, using either current technology or technology developed after the filing date of this patent, which would still fall within the scope of the claims.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

Additionally, certain embodiments are described herein as including logic or a number of routines, subroutines, applications, or instructions. These may constitute either software (e.g., code embodied on a non-transitory, machine-readable medium) or hardware. In hardware, the routines, etc., are tangible units capable of performing certain operations and may be configured or arranged in a certain manner. In example embodiments, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various embodiments, a hardware module may be implemented mechanically or electronically. For example, a hardware module may comprise dedicated circuitry or logic that may be permanently configured (e.g., as a special-purpose processor, such as a field programmable gate array (FPGA) or an application-specific integrated circuit (ASIC)) to perform certain operations. A hardware module may also comprise programmable logic or circuitry (e.g., as encompassed within a general-purpose processor or other programmable processor) that may be temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering embodiments in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules may provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connect the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it may be communicatively coupled. A further hardware module may then, at a later time, access the memory device to retrieve and process the stored output. Hardware modules may also initiate communications with input or output devices, and may operate on a resource (e.g., a collection of information).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods or routines described herein may be at least partially processor-implemented. For example, at least some of the operations of a method may be performed by one or more processors or processor-implemented hardware modules. The performance of certain of the operations may be distributed among the one or more processors, not only residing within a single machine, but deployed across a number of machines. In some example embodiments, the processor or processors may be located in a single location (e.g., within a home environment, an office environment, or as a server farm), while in other embodiments the processors may be distributed across a number of locations.

The performance of certain of the operations may be distributed among the one or more processors, not only residing within a single machine, but deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations.

Unless specifically stated otherwise, discussions herein using words such as "processing," "computing," "calculating," "determining," "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or a combination thereof), registers, or other machine components that receive, store, transmit, or display information.

As used herein any reference to "one embodiment" or "an embodiment" means that a particular element, feature, structure, or characteristic described in connection with the embodiment may be included in at least one embodiment. The appearances of the phrase "in one embodiment" in various places in the specification are not necessarily all referring to the same embodiment.

As used herein, the terms "comprises," "comprising," "may include," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description, and the claims that follow, should be read to include one or at least one and the singular also may include the plural unless it is obvious that it is meant otherwise.

This detailed description is to be construed as examples and does not describe every possible embodiment, as describing every possible embodiment would be impractical.

## Claims

1. A computer-implemented method of using machine learning to assess thermal endurance characteristics of materials, the computer-implemented method comprising:
training, by at least one processor, a machine learning model using a set of training data, wherein the set of training data (i) comprises training sets of coordinates indicative of a plurality of characteristics for a plurality of materials, and (ii) is labeled with at least one thermal endurance characteristic for each material of the plurality of materials, wherein the at least one thermal endurance characteristic is associated with each training set of coordinates, of the training sets of coordinates, for that material;
accessing, by the at least one processor, sets of coordinates indicative of the plurality of characteristics for a candidate material;
analyzing, by the at least one processor using the machine learning model that was trained, the sets of coordinates indicative of the plurality of characteristics for the candidate material; and
based on the analyzing, outputting, by the machine learning model, at least one predicted thermal endurance characteristic for the candidate material.

2. The computer-implemented method of claim 1, wherein the set of training data is labeled with at least one of an electrical relative thermal index (RTI), a mechanical impact RTI, or a mechanical strength RTI.

3. The computer-implemented method of claim 1, wherein training the machine learning model comprises:
training, by the at least one processor, the machine learning model using the set of training data and a set of validation data, wherein the set of validation data (i) comprises validation sets of coordinates indicative of the plurality of characteristics for the plurality of materials, and (ii) is labeled with the at least one thermal endurance characteristic for each material of the plurality of materials, wherein the at least one thermal endurance characteristic is associated with each validation set of coordinates, of the validation sets of coordinates, for that material.

4. The computer-implemented method of claim 1, wherein accessing the sets of coordinates indicative of the plurality of characteristics for the candidate material comprises:
accessing, by the at least one processor, the sets of coordinates indicative of at least one of: an infrared analysis, a thermogravimetric analysis, or a differential scanning calorimetry for the candidate material.

5. The computer-implemented method of claim 1, further comprising:
accessing, by the at least one processor, at least one actual thermal endurance characteristic for the candidate material; and
updating, by the at least one processor, the machine learning model with (i) the sets of coordinates indicative of the plurality of characteristics for the candidate material, and (ii) the at least one actual thermal endurance characteristic for the candidate material.

6. The computer-implemented method of claim 1, wherein outputting, by the machine learning model, the at least one predicted thermal endurance characteristic for the candidate material comprises:
outputting, by the machine learning model, (i) the at least one predicted thermal endurance characteristic for the candidate material and (ii) a confidence level for each of the at least one predicted thermal endurance characteristic for the candidate material.

7. The computer-implemented method of claim 1, wherein accessing the sets of coordinates indicative of the plurality of characteristics for the candidate material comprises:
accessing, by the at least one processor, the sets of coordinates, wherein each of the sets of coordinates comprises (x,y) coordinates indicative of a respective characteristic of the plurality of characteristics.

8. A system for using machine learning to assess thermal endurance characteristics of materials, comprising:
a memory storing a set of computer-readable instructions; and
one or more processors interfaced with the memory, and configured to execute the set of computer-readable instructions to cause the one or more processors to:
train a machine learning model using a set of training data, wherein the set of training data (i) comprises training sets of coordinates indicative of a plurality of characteristics for a plurality of materials, and (ii) is labeled with at least one thermal endurance characteristic for each material of the plurality of materials, wherein the at least one thermal endurance characteristic is associated with each training set of coordinates, of the training sets of coordinates, for that material,
access a set of coordinates indicative of the plurality of characteristics for a candidate material,
analyze, using the machine learning model that was trained, the set of coordinates indicative of the plurality of characteristics for the candidate material, and
based on the analyzing, output, by the machine learning model, at least one predicted thermal endurance characteristic for the candidate material.

9. The system of claim 8, wherein the set of training data is labeled with at least one of an electrical relative thermal index (RTI), a mechanical impact RTI, or a mechanical strength RTI.

10. The system of claim 8, wherein to train the machine learning model, the one or more processors is configured to:
train the machine learning model using the set of training data and a set of validation data, wherein the set of validation data (i) comprises validation sets of coordinates indicative of the plurality of characteristics for the plurality of materials, and (ii) is labeled with the at least one thermal endurance characteristic for each material of the plurality of materials, wherein the at least one thermal endurance characteristic is associated with each validation set of coordinates, of the validation sets of coordinates, for that material.

11. The system of claim 8, wherein the set of coordinates is indicative of at least one of:
an infrared analysis, a thermogravimetric analysis, or a differential scanning calorimetry for the candidate material.

12. The system of claim 8, wherein the one or more processors is configured to execute the set of computer-readable instructions to further cause the one or more processors to:
access at least one actual thermal endurance characteristic for the candidate material, and
update the machine learning model with (i) the set of coordinates indicative of the plurality of characteristics for the candidate material, and (ii) the at least one actual thermal endurance characteristic for the candidate material.

13. The system of claim 8, wherein the machine learning model outputs (i) the at least one predicted thermal endurance characteristic for the candidate material and (ii) a confidence level for each of the at least one predicted thermal endurance characteristic for the candidate material.

14. The system of claim 8, wherein each of the sets of coordinates comprises (x,y) coordinates indicative of a respective characteristic of the plurality of characteristics.

15. A non-transitory computer-readable storage medium configured to store instructions executable by one or more processors, the instructions comprising:
instructions for training a machine learning model using a set of training data, wherein the set of training data (i) comprises training sets of coordinates indicative of a plurality of characteristics for a plurality of materials, and (ii) is labeled with at least one thermal endurance characteristic for each material of the plurality of materials, wherein the at least one thermal endurance characteristic is associated with each training set of coordinates, of the training sets of coordinates, for that material;
instructions for accessing a set of coordinates indicative of the plurality of characteristics for a candidate material;
instructions for analyzing, using the machine learning model that was trained, the set of coordinates indicative of the plurality of characteristics for the candidate material; and
instructions for, based on the analyzing, outputting, by the machine learning model, at least one predicted thermal endurance characteristic for the candidate material.

16. The non-transitory computer-readable storage medium of claim 15, wherein the set of training data is labeled with at least one of an electrical relative thermal index (RTI), a mechanical impact RTI, or a mechanical strength RTI.

17. The non-transitory computer-readable storage medium of claim 15, wherein the instructions for training the machine learning model comprise:
instructions for training the machine learning model using the set of training data and a set of validation data, wherein the set of validation data (i) comprises validation sets of coordinates indicative of the plurality of characteristics for the plurality of materials, and (ii) is labeled with the at least one thermal endurance characteristic for each material of the plurality of materials, wherein the at least one thermal endurance characteristic is associated with each validation set of coordinates, of the validation sets of coordinates, for that material.

18. The non-transitory computer-readable storage medium of claim 15, wherein the instructions for accessing the set of coordinates indicative of the plurality of characteristics for the candidate material comprise:
instructions for accessing the set of coordinates indicative of at least one of: an infrared analysis, a thermogravimetric analysis, or a differential scanning calorimetry for the candidate material.

19. The non-transitory computer-readable storage medium of claim 15, wherein the instructions further comprise:
instructions for accessing at least one actual thermal endurance characteristic for the candidate material; and
instructions for updating the machine learning model with (i) the set of coordinates indicative of the plurality of characteristics for the candidate material, and (ii) the at least one actual thermal endurance characteristic for the candidate material.

20. The non-transitory computer-readable storage medium of claim 15, wherein the instructions for outputting, by the machine learning model, the at least one predicted thermal endurance characteristic for the candidate material comprise:
instructions for outputting, by the machine learning model, (i) the at least one predicted thermal endurance characteristic for the candidate material and (ii) a confidence level for each of the at least one predicted thermal endurance characteristic for the candidate material.
